# EUROPEAN PATENT APPLICATION

(11) **EP 4 238 628 A1**
(43) Date of publication of application: **06.09.2023**
(21) Application number: 23152737.5
(22) Date of filing: 20.01.2023
(51) Int. Cl.: B01D 46/00, A61L 9/20

(54) **INDUSTRIAL PURIFIER AND CENTRALIZED FILTRATION PLANT**

(30) Priority: 02.03.2022 IT 202200003854
(71) Applicant: Losma S.p.a., 24035 Curno (IT)
(72) Inventor: PADOVAN, Luca Emilio, 24020 PRADALUNGA BG (IT)
(74) Representative: Modiano, Micaela Nadia

(57) **Abstract**

An industrial purifier (1), comprising a box-like body (2) which defines a passage channel, and air suction means adapted to draw air from the outside environment and to convey it into the passage channel within which a first and a second air purification stage (3, 4) are arranged mutually in series;
the first purification stage (3) comprises first radiating means of ultraviolet light radiation which is adapted to strike the air flow drawn by the suction means for the sanitization thereof and in that the second purification stage (4) comprises photocatalytic oxidation means (6) for treating the previously sanitized air.

## Description

The present invention relates to an industrial purifier and a centralized filtration plant.

In the sector of disinfecting environments the use is known of ultraviolet light for air purification.

In more detail, this operation occurs by virtue of the fact that, from research carried out, ultraviolet light appears to be capable of at least partially reducing the viral load present in the air.

Thus industrial purifiers have been provided that are capable of emitting ultraviolet light for disinfecting environments.

These purifiers consist substantially of a form of lamp that emits ultraviolet rays, which is provided with a battery of ultraviolet LEDs that are controlled by dedicated electronics.

Such conventional purifiers are not devoid of drawbacks, among which there is the fact that they have limited filtering capacity, meaning air flow, and a limited inactivation level.

The aim of the present invention consists in providing an industrial purifier that is capable of offering higher performance than those of the known art.

Within this aim, an object of the present invention consists in providing an industrial purifier that is configured so that it can be implemented in a centralized filtration plant.

Another object of the present invention consists in providing an industrial purifier that is capable of offering the widest guarantees of reliability and safety in use.

Another object of the present invention consists in providing an industrial purifier that can be provided with technologies that are known per se and which therefore is low-cost.

This aim and these and other objects which will become more apparent hereinafter are achieved by an industrial purifier, comprising a box-like body which defines a passage channel, and air suction means adapted to draw air from the outside environment and to convey it into said passage channel; a first and a second air purification stage being comprised within said passage channel and being arranged mutually in series; characterized in that said first purification stage comprises first radiating means of ultraviolet light radiation which is adapted to strike the air flow drawn by said suction means for the sanitization thereof and in that said second purification stage comprises photocatalytic oxidation means for treating said previously sanitized air.

Further characteristics and advantages of the invention will become more apparent from the detailed description of a preferred, but not exclusive, embodiment of an industrial purifier, illustrated by way of nonlimiting example with the aid of the accompanying drawings wherein:
- Figure 1 is a perspective view of the industrial purifier according to the present invention;
- Figure 2 is a perspective view of the first purification stage of the industrial purifier shown in Figure 1;
- Figure 3 is a schematic plan view from above of the first purification stage shown in Figure 2 during its operation;
- Figure 4 is a first schematic side view of the first purification stage shown in Figure 2 during its operation;
- Figure 5 is a second schematic side view of the first purification stage shown in Figure 2 during its operation;
- Figure 6 is a perspective view of the second purification stage of the industrial purifier shown in Figure 1;
- Figure 7 is an exploded perspective view of the second purification stage shown in Figure 6;
- Figure 8 is a perspective view of the photocatalytic oxidation means of the second purification stage shown in Figure 7;
- Figure 9 is an exploded perspective view of the photocatalytic oxidation means shown in Figure 8;
- Figure 10 is a front elevation view of the photocatalytic oxidation means shown in the previous figures;
- Figure 11 is a front elevation view of the photocatalytic oxidation means shown in the previous figures, during its operation;
- Figure 12 is a side view of the photocatalytic oxidation means shown in the previous figures, during its operation;
- Figure 13 is a schematic view of a centralized filtration plant according to the invention;
- Figures 14 and 15 are two block diagrams representing the operation of the plant shown in Figure 13.

With particular reference to the figures, the industrial purifier according to the invention, generally designated by the reference numeral 1, comprises a box-like body 2 which defines a passage channel, and air suction means adapted to draw air from the outside environment and to convey it into said passage channel.

In the embodiment proposed, since the geometries of the passage channel and the air suction means are, respectively, at the complete discretion of the designer and are known per se, they are not shown in the figures.

Conveniently, a first and a second air purification stage 3 and 4 are comprised within the passage channel and are arranged mutually in series.

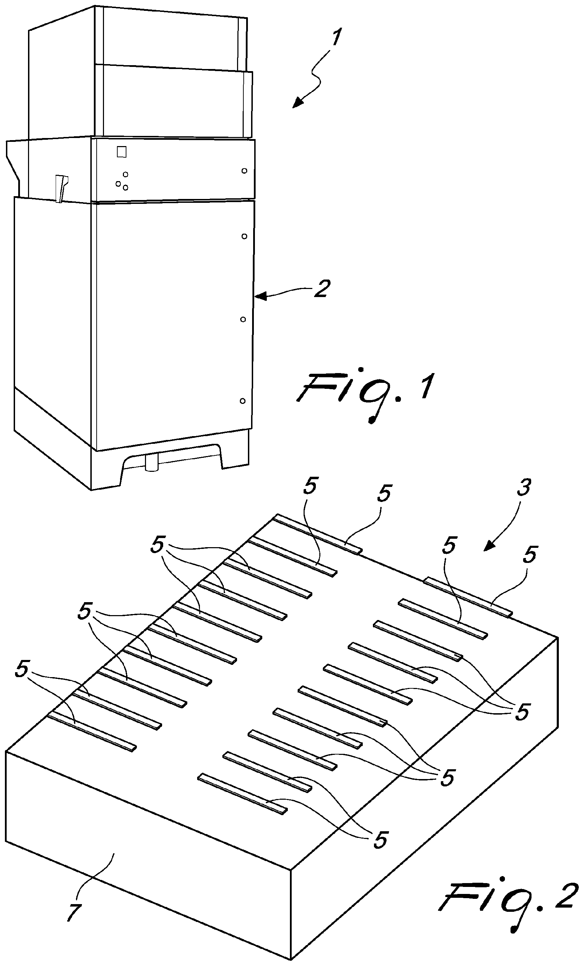

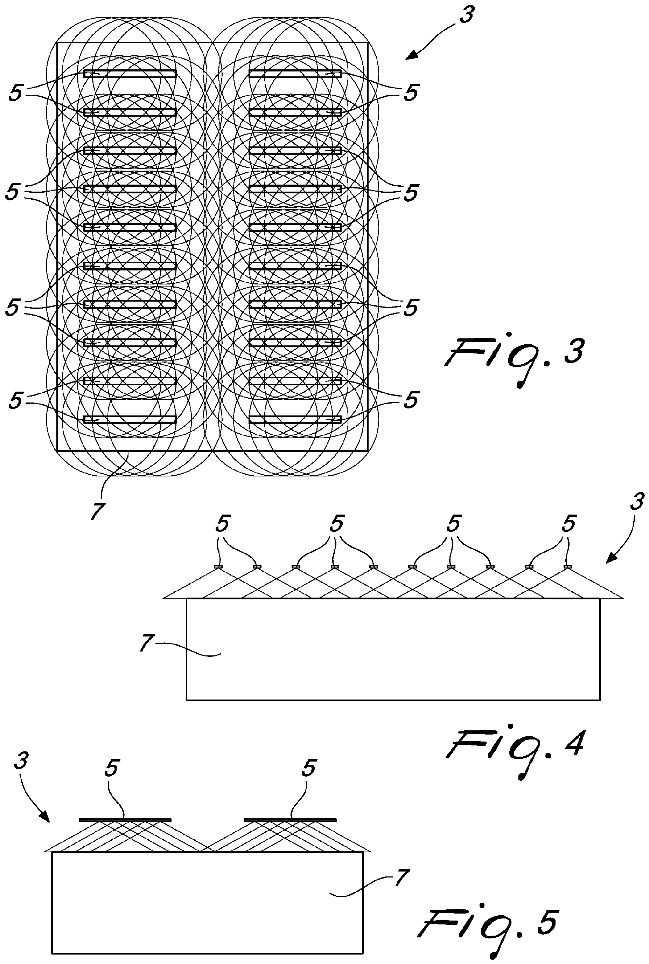

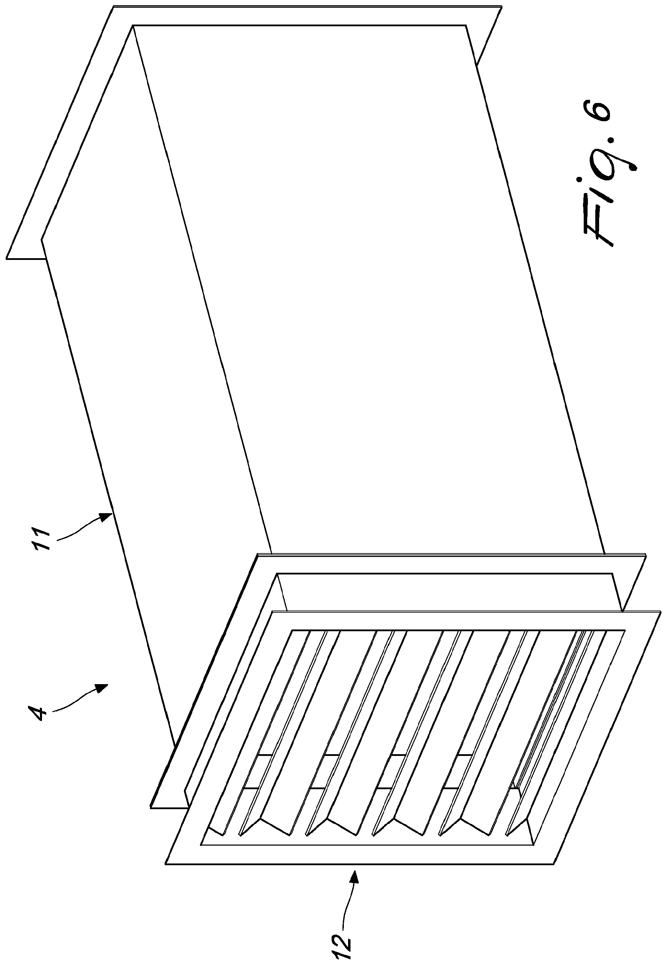

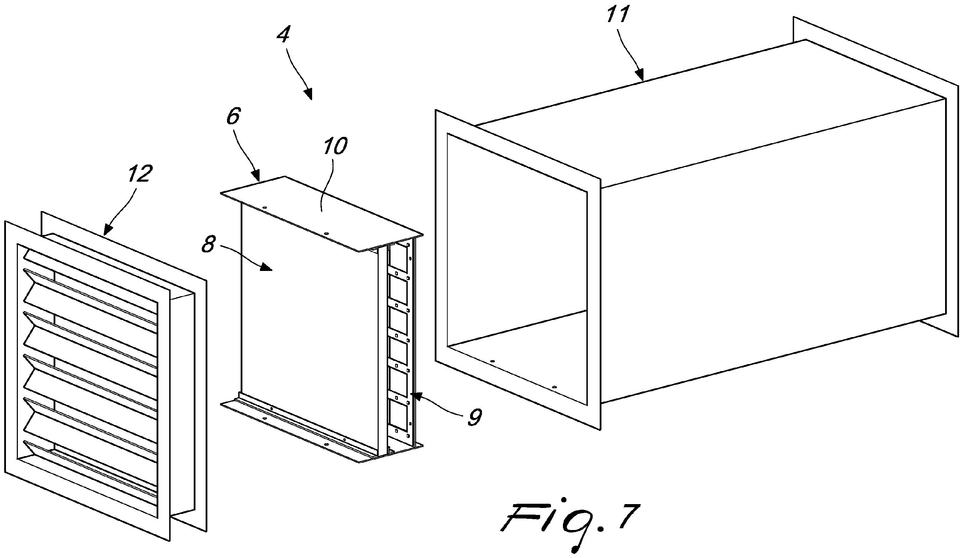

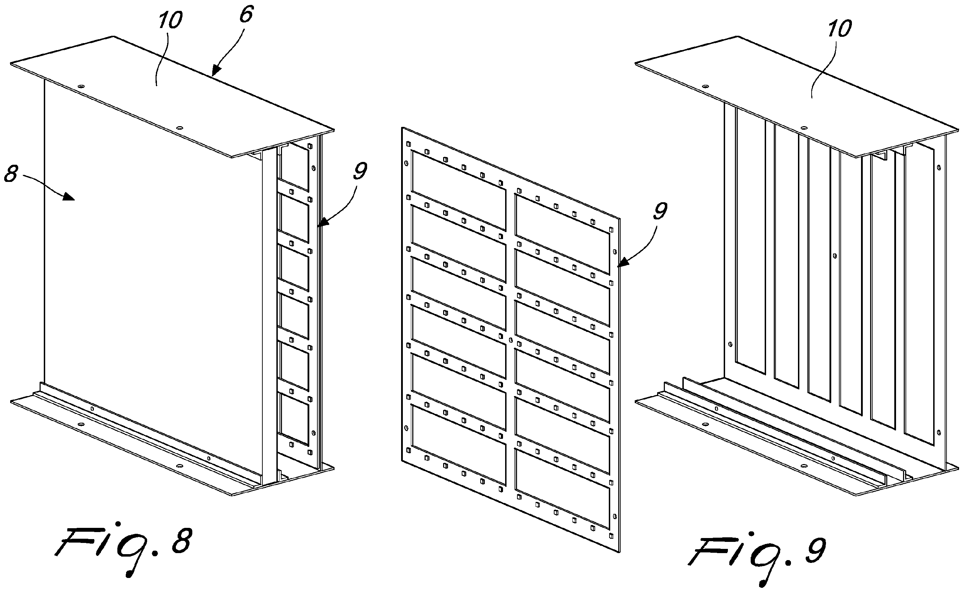

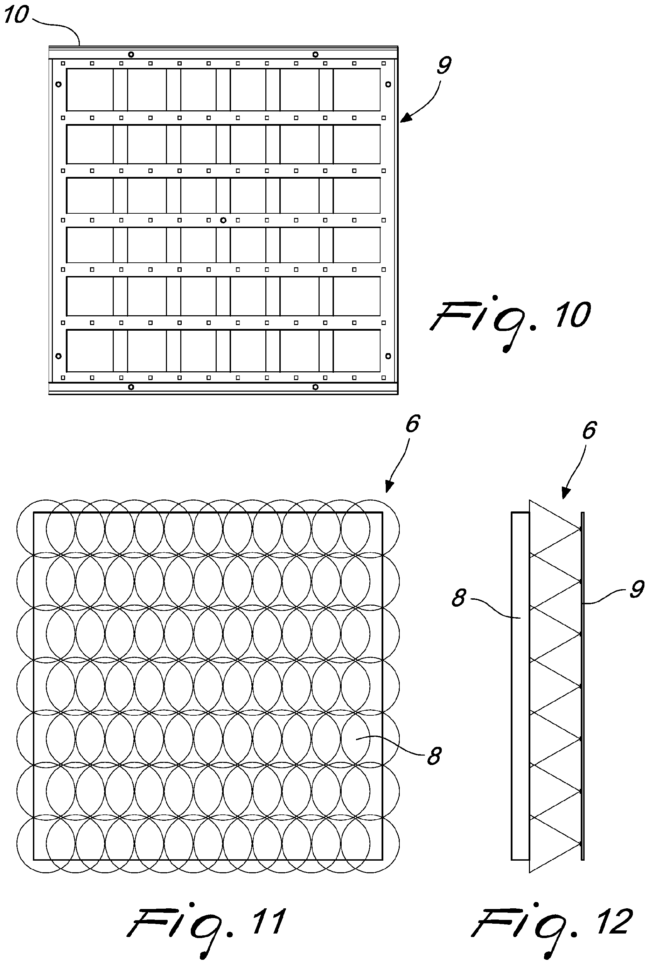

According to the invention, the first purification stage 3, which is shown in detail in Figures 2 to 5, comprises first radiating means 5 of ultraviolet light radiation which is adapted to strike the air flow drawn by the suction means for the sanitization thereof, and the second purification stage 4, which is shown in detail in Figures 6 to 12, comprises photocatalytic oxidation means 6 for treating the air that was previously sanitized in the first purification stage 3.

In more detail, the first radiating means 5 comprise a plurality of UV-C LED modules deployed against the current with respect to the direction of the air flow passing through the passage channel, and these are configured so as to irradiate the filtering surface of an absolute filter 7 with which the suction means are fitted.

In fact, these suction means can comprise an industrial suction unit fitted with the absolute filter 7 so as to filter the air of aerosols, solid particulate matter, and vapors with a degree of filtration of H13 (i.e., efficiency equal to 99.97%).

This particular arrangement of the UV-C LED modules, arranged at the outlet of the absolute filter 7 and against the current with respect to the air flow, makes it possible to maximize the efficiency of sanitization of the system.

After being filtered to a degree of filtration of H13, the air undergoes a first sanitization treatment using UV-C radiation which will damage the DNA of virus and bacteria cells.

Furthermore, the penetrating action of the UV-C rays makes it possible to sanitize the filtering surface of the absolute filter 7 in depth, an aspect of fundamental importance during the step of maintenance/substitution of the absolute filter 7 in that the operator can perform their work with a low risk of microbiological contamination.

Furthermore, the UV-C LED modules, which can number twenty for example, can develop a high power equal to 134 watts and this power is managed by an electronic control system which, using sophisticated firmware, ensures the automatic execution of several functions:
- power modulation of the UV-C LEDs using PWM (Pulse Width Modulation) logic, which maximizes the effectiveness of the damage to virus and bacteria cells;
- "intelligent" power modulation makes it possible to reduce energy consumption and to extend the useful life of the LED modules beyond 24,000 hours of operation;
- a self-diagnostic system, which continuously monitors the status of operation of the UV-C LED modules, reporting any anomalies;
- managing the LED modules UV-C in safety during the steps of maintenance of the purification system.

Considering the photocatalytic oxidation means 6, these comprise a PCO (Photocatalytic Oxidation) filter 8 and second radiating means 9 of ultraviolet light radiation which strikes the PCO filter 8.

In more detail, the PCO filter 8 is constituted by a ceramic cell coated with a titanium dioxide layer (TiO₂).

Advantageously, the second radiating means 9 of the second purification stage 4 comprise a plurality of UV-A LEDs oriented in such a manner as to irradiate the titanium dioxide (TiO₂) layer of the ceramic cell and activate the photocatalytic oxidation process.

In the embodiment proposed, the PCO filter 8 has been dimensioned to purify an air flow rate equal to 2,000 m³/h and the UV-A LEDs are associated with a PCB that controls the operation thereof.

Conveniently, the supporting frame 10 of the second radiating means 9 is configured to even out the air flow and simultaneously to ensure a uniform distribution of the UV-A radiation on the surface of the ceramic cell.

In more detail, the supporting frame 10 is of the grid type so that the aspirated air passes through the mesh of such grid, thus striking and subsequently filtering through the PCO filter 8 irradiated by the UV-A LEDs.

Furthermore, each individual UV-A LED is characterized by a wavelength equal to 365nm and by a power of 2.5 watts; the PCB (Printed Circuit Board) therefore needs to handle an overall power of 210 watts, corresponding to eighty-four UV-A LEDs.

Such power is managed by a specific electronic control system (a driver) which is capable of regulating the power supply of the UV-A LEDs and of monitoring the temperature of the PCB using a specific temperature probe, thus making it possible to reduce energy consumption and extend the useful life of the LEDs beyond 35,000 hours of operation, and, via an indicator light, the driver is also capable of monitoring the status of operation of the electronic system (ON/OFF logic), reporting any anomalies (for example high operating temperature).

Conveniently, the photocatalytic oxidation means 6 are associated with a tubular duct 11, through which the aspirated air flows inward, and the PCO filter 8 with the PCB, which are associated with the supporting frame 10, are arranged at the outlet of the tubular duct 11 and the supporting frame 10 supports a PCB for the UV-ALEDs.

Furthermore, an adjustment shutter 12 is comprised which is arranged so as to close the tubular duct 11 and is adapted to convey said treated air toward the outside environment.

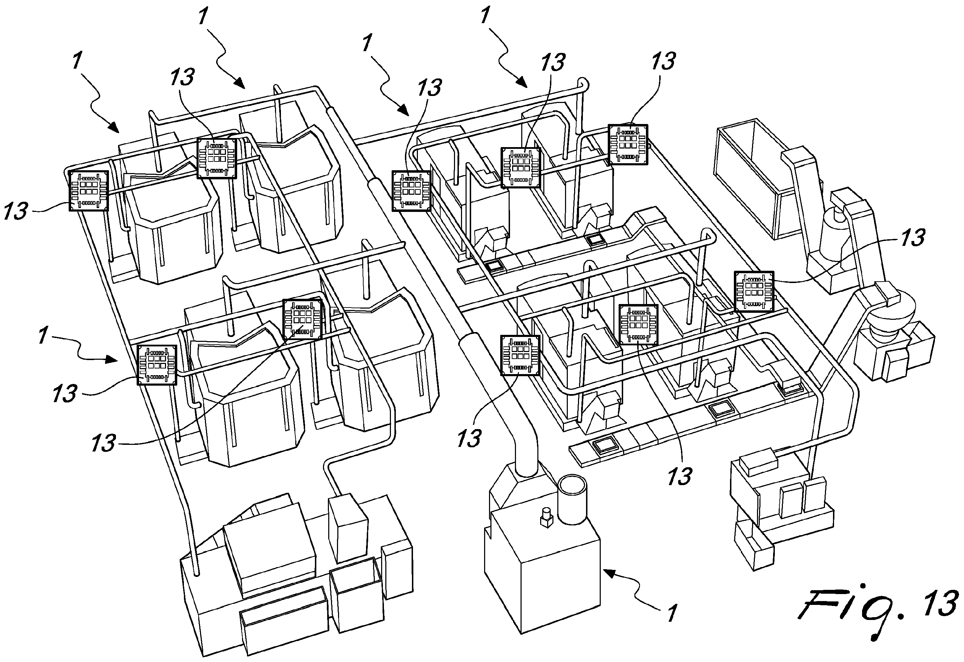

The industrial purifier 1 can be integrated in a centralized filtration plant, generally designated by the reference numeral 100 in Figure 13.

In more detail, the industrial purifier 1 is functionally associated with a logic control unit 14 (PID) and there are electronic sensor means 13 each of which is located in a specific area to be controlled and is functionally associated with the logic control unit so as to continuously monitor the air quality level and therefore automatically manage the centralized filtration plant 100.

In this way, it is possible to ensure a high and constant level of healthfulness in the industrial environment.

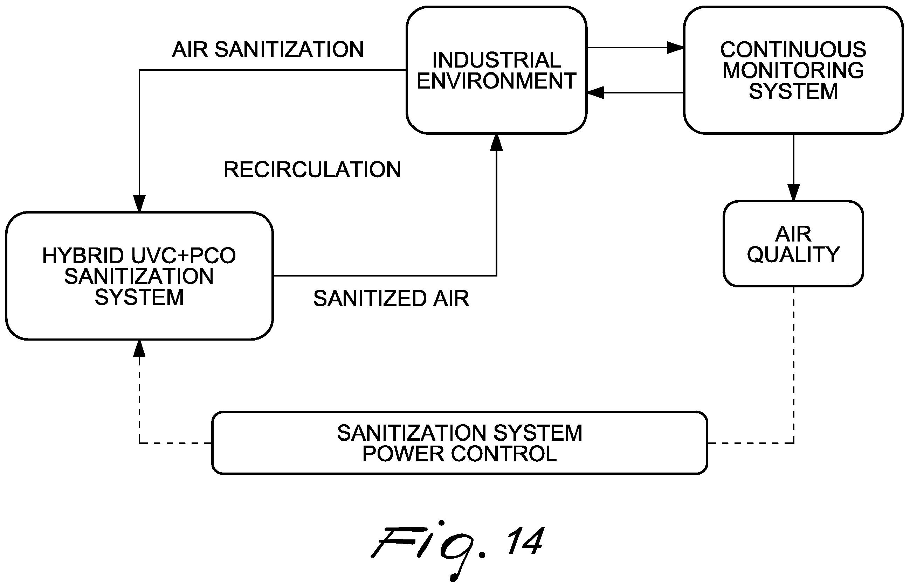

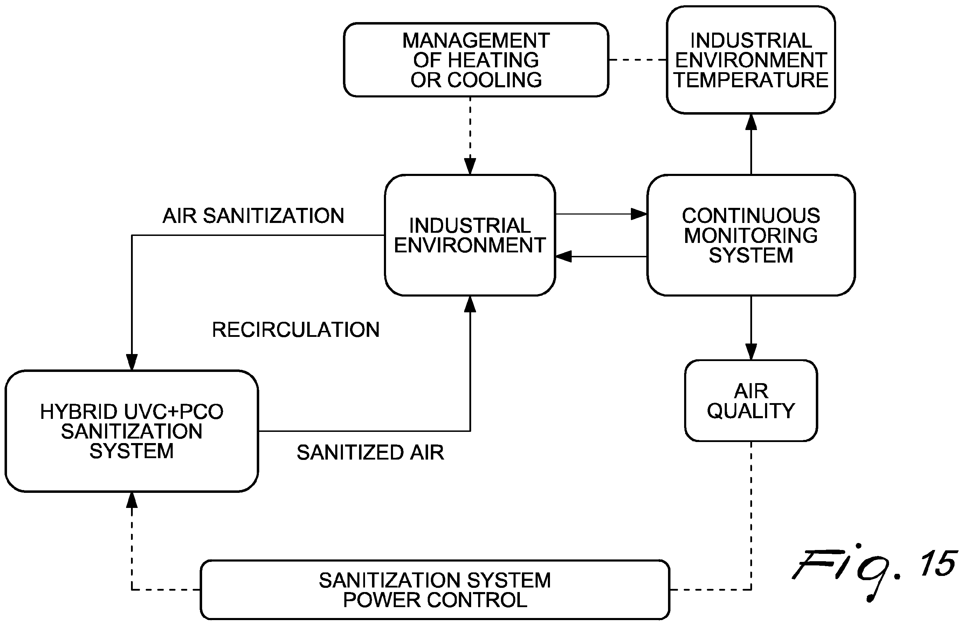

With particular reference to Figures 14 and 15, it is possible to define the control logic of the sanitization process via the continuous monitoring of the air quality in the industrial environment.

In practice it has been found that the industrial purifier achieves the intended aim and objects.

In fact, the hybrid technology of UVC+PCO (photocatalytic oxidation) is capable of ensuring air purified of viruses, bacteria, VOCs, NOx, dust, allergens, odors and mildew in an industrial environment.

Such technology is capable of operating in centralized plants for filtering air and therefore of treating significant volumes of air, up to 30,000 m³/h.

Furthermore, this technology is capable of abating the SARS-COV-2 virus with an abatement coefficient equal to LOG 4, corresponding to an inactivation level of 99.99% of infective load.

The innovation of this technology consists in the synergistic use of UVC and PCO technologies, and of continuously and automatically controlling the operation of the entire purification system.

With this hybrid technology it is possible to reach high levels of quality and of healthfulness of the air inside the working environment.

More specifically, the automated recirculation of air, managed by the control logic described above, makes it possible to recover heat energy both during heating (in winter) and during cooling (in the summer), which otherwise would be dissipated in the atmosphere if there was no recirculation system.

Differently, in a traditional centralized industrial filtration system, the air is filtered down to a degree of filtration of F9 and subsequently is released into the atmosphere with a certain energy content (dissipated heat energy).

By virtue of the monitoring system, however, it is possible to monitor the internal temperature of the working environment and then control the step of heating or cooling in the industrial plant more effectively.

This enables a considerable energy saving and a reduction of approximately 35% of CO₂ emissions into the atmosphere, deriving from the combustion of methane gas or the use of electricity produced using fossil fuels.

The industrial purifier, thus conceived, is susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102022000003854 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An industrial purifier (1), comprising a box-like body (2) which defines a passage channel, and air suction means adapted to draw air from the outside environment and to convey it into said passage channel; a first and a second air purification stage (3, 4) being comprised within said passage channel and being arranged mutually in series; **characterized in that** said first purification stage (3) comprises first radiating means of ultraviolet light radiation which is adapted to strike the air flow drawn by said suction means for the sanitization thereof and **in that** said second purification stage (4) comprises photocatalytic oxidation means (6) for treating said previously sanitized air.

2. The industrial purifier (1) according to claim 1, **characterized in that** said suction means comprise an industrial suction unit provided with an absolute filter (7) so as to filter the air of aerosols, solid particulate matter, and vapors.

3. The industrial purifier (1) according to claim 2, **characterized in that** said first radiating means of said first purification stage (3) are configured in such a manner as to irradiate the filtering surface of said absolute filter (7).

4. The industrial purifier (1) according to one or more of the preceding claims, **characterized in that** said first radiating means (5) of said first purification stage (3) comprise a plurality of UV-C LED modules deployed against the current with respect to the direction of the air flow that passes through said passage channel.

5. The industrial purifier (1) according to one or more of the preceding claims, **characterized in that** said photocatalytic oxidation means (6) comprise a PCO filter (8) and second radiating means (9) of ultraviolet light radiation which strikes said PCO filter (8).

6. The industrial purifier (1) according to claim 5, **characterized in that** said PCO filter (8) is constituted by a ceramic cell coated with a titanium dioxide layer.

7. The industrial purifier (1) according to claim 6, **characterized in that** said second radiating means (9) of said second purification stage (4) comprise a plurality of UV-A LEDs oriented in such a manner as to irradiate said titanium dioxide layer of said ceramic cell and activate the photocatalytic oxidation process.

8. The industrial purifier (1) according to claim 7, **characterized in that** it comprises a tubular duct (11) which is associated with said photocatalytic oxidation means (6) and through which said aspirated air flows inward; said PCO filter (8) being associated with a supporting frame (10) which is arranged at the outlet of said tubular duct (11) and which supports a PCB for said UV-A LEDs; said supporting frame (10) being of the grid type so that said aspirated air passes through the mesh of said grid, thus striking and subsequently filtering through said PCO filter (8) irradiated by said UV-A LEDs; an adjustment shutter (12) being further comprised which is arranged so as to close said tubular duct (11) and is adapted to convey said treated air toward the outside environment.

9. A centralized filtration plant (100), **characterized in that** it comprises at least one industrial purifier (1) according to one or more of the preceding claims, which is functionally associated with a logic control unit, and **in that** it comprises electronic sensor means (13) which are functionally associated with said logic control unit so as to continuously monitor the air quality level and therefore automatically manage said centralized filtration plant (100) so as to ensure a high and constant level of healthfulness in the industrial environment; said electronic sensor means (13) being installed proximate to specific areas to be controlled.
